(19) Europäisches Patentamt
European Patent Office
Office européen des brevets

(11) Veröffentlichungsnummer: **0 392 462 B1**

# (12) EUROPÄISCHE PATENTSCHRIFT

(45) Veröffentlichungstag der Patentschrift: **15.03.95**

(51) Int. Cl.[6]: **G01N 31/22**, G01N 33/52

(21) Anmeldenummer: **90106851.0**

(22) Anmeldetag: **11.04.90**

## (54) Verfahren zur selektiven komplexometrischen quantitativen Bestimmung von N-(Phosphonomethyl)-glycin-(glifosat(R)) und Derivat davon.

(30) Priorität: **11.04.89 HU 172489**

(43) Veröffentlichungstag der Anmeldung:
**17.10.90 Patentblatt 90/42**

(45) Bekanntmachung des Hinweises auf die Patenterteilung:
**15.03.95 Patentblatt 95/11**

(84) Benannte Vertragsstaaten:
**BE DE ES GB**

(56) Entgegenhaltungen:

**J. AGRIC. FOOD. CHEM., Band 32, 1984, Seiten 1249-1253, American Chemical Society, Washington, DC, US; R.L. GLASS: "Metal complex formation by glyphosate"**

**J. AGRIC. FOOD CHEM., Band 36, 1988, Seiten 1326-1329, American Chemical Society, Washington, DC, US; V. SUBRAMANIAM et al.: "Metal complexes of glyphosate"**

(73) Patentinhaber: **MONSANTO EUROPE S.A.**
**Avenue de Tervuren 270-272**
**Letter Box 1**
**B-1150 Brussels (BE)**

(72) Erfinder: **Repasi, Janos, Dr.**
**4440 Tiszavasvari**
**1/4 ifjusag u (HU)**

(74) Vertreter: **Beszédes, Stephan G., Dr.**
**Patentanwalt**
**Postfach 11 68**
**D-85201 Dachau (DE)**

EP 0 392 462 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur selektiven komplexometrischen quantitativen Bestimmung von N-(Phosphonomethyl)-glycin, N-(Phosphonomethyl)-N-(carboxy-methyl)-glycin, N,N-bis-(Phosphonomethyl)-glycin oder einer Mischung von N-(Phosphonomethyl)-glycin und N-(Phosphonomethyl)-N-(carboxy-methyl)-glycin oder einer Mischung von N-(Phosphonomethyl)-glycin und N,N-bis-(Phosphonomethyl)-glycin.

N-(Phosphonomethyl)-glycin ist ein Herbicid mit breitem Wirkungsspektrum, welches im Jahre 1971 auf den Markt gebracht wurde (Handelsname: Glyphosat®, Hersteller: Monsanto). In den vergangenen Jahren bestätigten sich seine außerordentlich vorteilhaften Eigenschaften. Seine Alkalimetallsalze und Salze mit aliphatischen Aminen sind sehr gut wasserlöslich. Es ist praktisch nicht toxisch (oraler $LD_{50}$-Wert an Ratten: 4 300 mg/kg). Bei seiner vorschriftsmäßigen Verwendung ist keine arbeitshygienische und ernährungshygienische Wartezeit erforderlich. Seine Wirkungsdauer ist sehr lang. Mit einem Spritzen im Herbst kann ein 6 bis 8 Monate dauerndes Freisein von Unkraut erreicht werden. Es ist gegen die meisten Süßgräser in Aufwandmengen von 1 bis 4 kg/ha und gegen 1-jährige Unkräuter schon in Aufwandmengen von 0,5 bis 1 kg/ha wirksam. Es ist eine umweltschonende Verbindung. Im Boden wird es schnell gebunden und wandelt sich hauptsächlich durch mikrobiologische Zersetzung zu Naturstoffen, wie Kohlendioxyd, Ammoniak und Phosphate, um. Wegen dieser Eigenschaften wächst die Nachfrage nach diesem Mittel von Jahr zu Jahr und heute ist es eines der auf der Welt in den größten Mengen verwendeten Herbicide.

Die Herstellung dieses Produktes ist durch zahlreiche Patente geschützt. Von diesen verbreiteten sich in der Technik praktisch 2 grundlegende Verfahren, deren Reaktionsschemen im folgenden wiedergegeben sind.

a)

b)

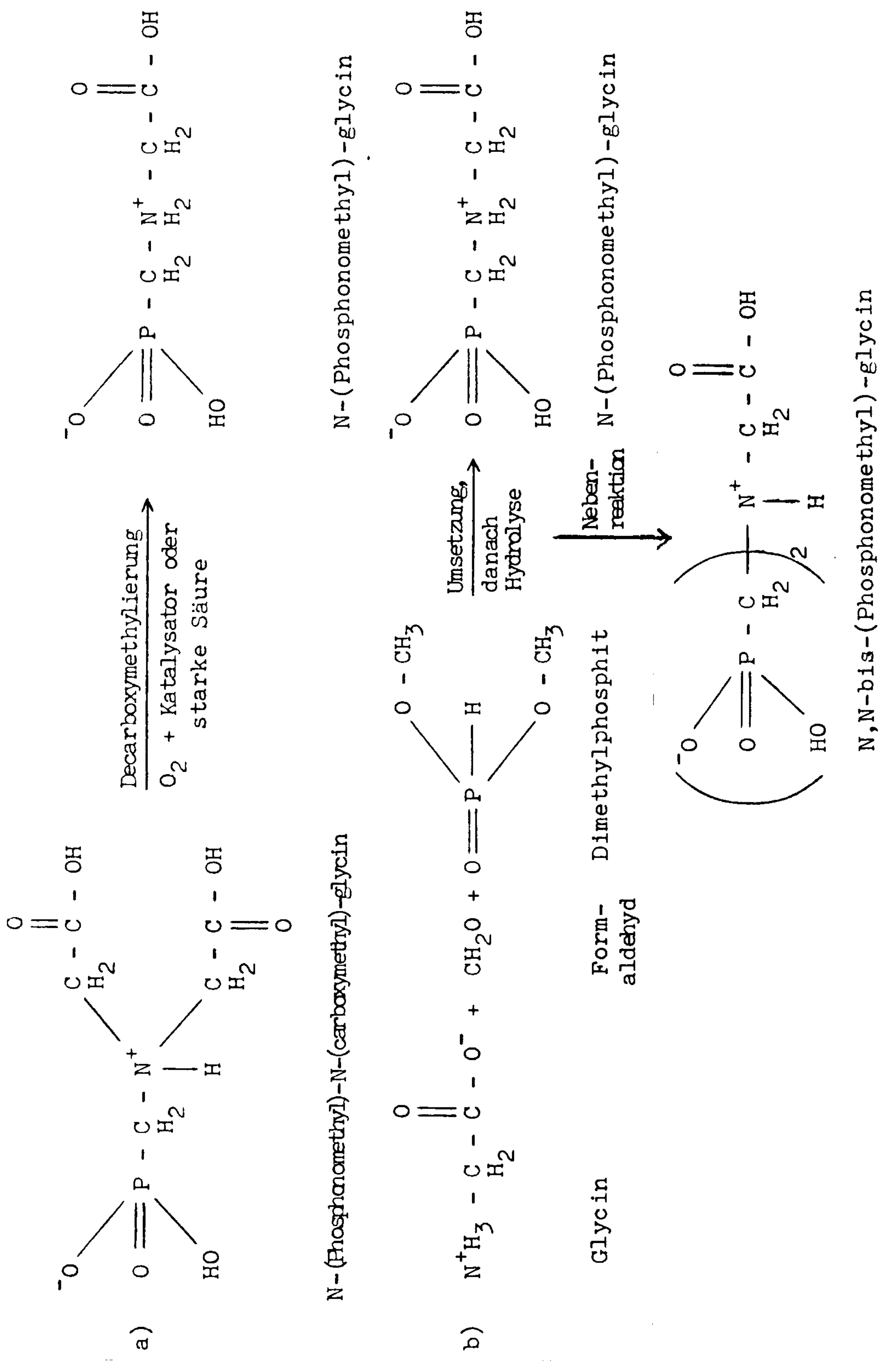


N-(Phosphonomethyl)-N-(carboxymethyl)-glycin

N-(Phosphonomethyl)-glycin

Glycin

Form-aldehyd

Dimethylphosphit

N-(Phosphonomethyl)-glycin

N,N-bis-(Phosphonomethyl)-glycin

Bei der Umsetzung a) ist das Verfolgen der Umsetzung sehr wichtig. Wenn nämlich das Verfahren zu früh beendet wird, dann verbleibt eine bedeutende Menge N-(Phosphonomethyl)-N-(carboxy-methyl)-glycin im Endprodukt. Wenn dagegen das Verfahren zu spät beendet wird, dann ist mit einer Weiterzersetzung des N-(Phosphonomethyl)-glycines zu Aminomethylphosphonsäure zu rechnen.

Beim Verfahren b) müssen die Kondensationsparameter sehr streng eingehalten werden, da sonst die dargestellte Nebenreaktion beschleunigt wird und viel N,N-bis-(Phosphonomethyl)-glycin erscheint. Übrigens ist diese Verbindung als den Zuckergehalt erhöhendes Mittel auch an sich gebräuchlich (Polaris, Monsanto). Daher ist auch die Bestimmung des zu diesem Zwecke hergestellten technischen N,N-bis-(Phosphonomethyl)-glycines und N,N-bis-(Phosphonomethyl)-glycinesin Zubereitung erwünscht.

Es sind zahlreiche Verfahren zur Bestimmung von N-(Phosphonomethyl)-glycin, N-(Phosphonomethyl)-N-(carboxy-methyl)-glycin und N,N-bis-(Phosphonomethyl)-glycin bekannt. Von mehreren Verfassern wurden magnetische kernresonanzspektroskopische (NMR-spektroskopische) Verfahren zur Untersuchung des Oxydationsgemisches von N-(Phosphonomethyl)-N-carboxy-methyl)-glycin angegeben. Diese können natürlich nicht als Routineverfahren angewandt werden.

Durch Nitrosieren in einem sauren Medium kann das als sekundäres Amin reagierende N-(Phosphonomethyl)-glycin mittels UV-Nachweis (Young, J. C. Khan, S. U.: J. Environ. Sci. Health B 13(1), 59 [1978]) oder Polarographie (Bronstad, J. O., Friestad, H. O.: Analyst 101 [1976], 820) gut bestimmt werden. Von Ekstron und Johanson wurde zur Bestimmung von N-(Phosphonomethyl)-glycin ein Aminosäure-Reagenz mittels Ninhydrin-Reaktion verwendet. Mit diesen Verfahren wird keine Information über den Gehalt der Proben an N-(Phosphonomethyl)-N-(carboxy-methyl)-glycin oder N,N-bis-(Phosphonomethyl)-glycin erhalten.

Im Falle von reinen Substanzen ist auch die potentiometrische Titration anwendbar, in welchen Falle aus dem Abweichen der Verhältnisse der potentiometrischen Stufen von 1 auf den N-(Phosphonomethyl)-N-(carboxy-methyl)-glycin- oder N,N-bis-(Phosphonomethyl)-glycin-Gehalt des N-(Phosphonomethyl)-glycines geschlossen werden kann. Natürlich führt dies im Falle von Reaktionsgemischen oder technischen Produkten zu völlig falschen Ergebnissen.

Die gaschromatographischen Bestimmungen werden dadurch erschwert, daß keines von N-(Phosphonomethyl)-glycin, N-(Phosphonomethyl)-N-(carboxy-methyl)-glycin und N,N-bis-(Phosphonomethyl)-glycin flüchtig ist. Durch Derivatbildung (Trifluoracetylieren und Methylieren mittels Diazomethan) kann diesen Problem ausgewichen werden, dadurch ist jedoch das Verfahren sehr schwerfällig und langwierig und daher ist es nur lohnend, es durch Untersuchung von Resten beziehungsweise Rückständen des Mittels anzuwenden.

Mehrere Verfasser befassen sich mit der HPLC-Analyse (Lundgren, L. N.: J. Agric. Food. Chem. 34 [1986], 535; Areher, T. E. und Mitarbeiter: J. Agric. Food. Chem. 32 [1984], 586). Da stellt die außerordentlich geringe UV-Absorption der Veroindungen die Schwierigkeit dar. Zwar ist die Vor- oder Nachkolonnen-Derivatbildung lösbar, aber auch in diesen Falle kommt das bei der Erörterung der Gaschromatographie Beschriebene zur Geltung.

Die Veröffentlichung von R. L. Glass: J. Agric. Food Chem., 32, 1984, Seiten 1249 bis 1253, befaßt sich mit der Herstellung von einigen Metallkomplexen von Glyphosat bzw. deren analytischer Untersuchung durch Polarographie, UV- und IR-Spektrophotometrie und HPLC. Sie befaßt sich aber nicht mit der analytischen Bestimmung von Glyphosat, vielmehr werden dort nur einige Metallion-Glyphosat-Komplexe hergestellt und dann deren Eigenschaften und physikalisch-chemische Parameter beschrieben.

Bekannt ist auch die kapillare Isotachophorese-Untersuchung der Reaktionsgemische (Krivankova, L., Bocek, P.: Electrophoresis, Weinheim, FRG., 7(2), 100 [1986]), aber auch dieses Verfahren kann nicht als Routineverfahren bezeichnet werden.

In Anbetracht des Obigen bestand ein Bedarf an einem solchen einfach und mit geringem Aufwand durchführbarem selektiven Verfahren, mit welchem N-(Phosphonomethyl)-glycin, N-(Phosphonomethyl)-N-(carboxy-methyl)-glycin und N,N-bis-(Phosphonomethyl)-glycin bestimmt werden können.

Die Komplexbildner-Eigenschaft der genannten Verbindungen wurde von mehreren Verfassern untersucht.

Von Schwarzenbach und Mitarbeitern (Helv. Chim. Acta 32 [1949], 1 175 wurden mehrere Iminodiessigsäure-Derivate verglichen und die Stabilitätskonstanten von einigen Metall-N-(Phosphonomethyl)-glycin-Komplexen mitgeteilt. Von Martell und Ockerbloom (J. Am. Chem. Soc. 80 [1958] 2 351) wurde auch die mögliche Struktur der Komplexe angegeben.

Von Fiedelman (US-PS 3 385 675) wurde die sehr schlechte Löslichkeit des N-(Phosphonomethyl)-N-(carboxy-methyl)-glycin-Calcium-Komplexes zum Befreien von Natriumchlorid von Calcium ausgenutzt.

Von Shopron und Sirkij (Koord. Khim. 2 [1976], 1 082) wurde die Elektronenverteilung des N-(Phosphonomethyl)-N-(carboxy-methyl)-glycines untersucht.

Hayasida (japanische Patentschrift 71 216 047) erreichte mit dem N-(Phosphonomethyl)-N-(carboxy-methyl)-glycin in einem alkalischen galvanischen Zinkbad das Inlösunghalten des Zinks.

Auf den Komplexbildnercharakter des N-(Phosphonomethyl)-glycines weist die Tatsache, daß die Wirksamkeit des Herbicides in Gegenwart von Eisen- und Calciumionen sich verringert und diese Wirksamkeitsverringerung durch Zugabe von Äthylendiamintetraessigsäure (EDTA) aufgehoben werden kann (Anonym: Res. Discl. 148 [1976], 10).

Mehrere Verfasser befaßten sich auch mit der Bestimmung der Stabilitätskonstanten. Einige charakteristische Werte (log $K_s$) von Metallkomplexen der obigen Verbindungen sind in der folgenden Tabelle 1 zusammengestellt.

Tabelle 1

| Komplexbildner-Metall | Stabilitätskonstanten log $K_s$ | | |
|---|---|---|---|
| | N-Phosphonomethyl)-glycin | N,N-bis-(Phosphonomethyl)-glycin | N-(Phosphonomethyl)-N--(carboxy-methyl)-glycin |
| Mg | 3,3 | 4,2 | 6,0 |
| Ca | 3,2 | 3,8 | 7,1 |
| Mn | 5,5 | 6,3 | 8,0 |
| Zn | 8,7 | 9,0 | - |
| Cu | 11,9 | 12,8 | 12,5 |

Der Erfindung liegt die Aufgabe zugrunde, ein einfach und mit geringem Aufwand durchführbares Verfahren zur quantitativen selektiven Bestimmung von N-(Phosphonomethyl)-glycin, N-(Phosphonomethyl)-N-(carboxy-methyl)-glycin, N,N-bis-(Phosphonomethyl)-glycin oder eines Gemisches von N-(Phosphonomethyl)-glycin und N-(Phosphonomethyl)-N-(carboxy-methyl)-glycin oder N-(Phosphonomethyl)-glycin und N,N-bis-(Phosphonomethyl)-glycin zu schaffen.

Das Obige wurde überraschenderweise durch die Erfindung erreicht.

Es wurde nämlich überraschenderweise festgestellt, daß die quantitative komplexometrische Bestimmung in Falle eines Metallionen-Indikator-pH-Systemes zur selektiven quantitativen Bestimmung von N-(Phosphonomethyl)-glycin und N-(Phosphonomethyl)-N-(carboxy-methyl)-glycin beziehungsweise N-(Phosphonomethyl)-glycin und N,N-bis-(Phosphonomethyl)-glycin nebeneinander geeignet ist.

Gegenstand der Erfindung ist daher ein Verfahren zur selektiven quantitativen Bestimmung von N-(Phosphonomethyl)-glycin, N-(Phosphonomethyl)-N-(carboxy-methyl)-glycin, N,N-bis-(Phosphonomethyl)-glycin beziehungsweise einer Mischung von N-(Phosphonomethyl)-N-(carboxy-methyl)-glycin und N-(Phosphonomethyl)-glycin oder einer Mischung von N-(Phosphonomethyl)-glycin und N,N-bis-(Phosphonomethyl-glycin, welches dadurch gekennzeichnet ist, daß

a) mit einer Wismut-Maßlösung der Gehalt an N-(Phosphonomethyl)-N-(carboxy-methyl)-glycin beziehungsweise N,N-bis-(Phosphonomethyl)-glycin bei einen pH-Wert von 1,5 bis 2,5, insbesondere 1,8 bis 2,5, in Gegenwart eines Methylthymolblau-Indikators durch komplexometrische Titration bestimmt wird und

b) mit einer Kupfer-Maßlösung die Summe des Gehaltes an N-(Phosphonomethyl)-glycin und N-(Phosphonomethyl)-N-(carboxy-methyl)-glycin oder N-(Phosphonomethyl)-glycin und N,N-bis-(Phosphonomethyl)-glycin bei einem pH-Wert von 8 bis 10 in Gegenwart eines Murexidindikators durch komplexometrische Titration bestimmt wird

sowie gegebenenfalls aus den Ergebnissen beider Bestimmungen der Gehalt der Probe an N-(Phosphonomethyl)-glycin berechnet wird.

Wie bereits gesagt, ist ein großer Vorteil des erfindungsgemäßen Verfahrens, daß N-(Phosphonomethyl)-glycin und N-(Phosphonomethyl)-N-(carboxy-methyl)-glycin beziehungsweise N-(Phosphonomethyl)-glycin und N,N-bis-(Phosphonomethyl)-glycin nebeneinander selektiv und quantitativ bestimmbar sind.

Ein weiterer Vorteil des erfindungsgemäßen Verfahrens ist, daß es mit geringem Aufwand durchführbar ist und keinen Apparat benötigt, sondern nur wenige grundlegende Chemikalien und Laboratoriumsglasgeräte. So ist das Verfahren auch auf einem solchen Gebiet anwendbar, auf welchem sonst für eine analytische Kontrolle keine Möglichkeit bestünde. Es eröffnet sich die Möglichkeit dazu, daß der Käufer den Wirkungsgrad des zubereiteten Mittels kontrolliert, beziehungsweise erforderlichenfalls auch die Konzentration der Spritzbrühe bestimmbar ist.

Die Schnelligkeit des Verfahrens macht es auch zum Verfolgen der Reaktion, im besonderen Hinblick auf die Herstellung des N-(Phosphonomethyl)-glycines aus dem N-(Phosphonomethyl)-N-(carboxy-methyl)-glycin geeignet.

Vorzugsweise wird als Wismut-Maßlösung eine basische Wismutnitratlösung in Salpetersäure verwendet. Insbesondere wird als basische Wismutnitratlösung eine solche mit einer Konzentration von 0,01 bis

0,02 Mol/l, ganz besonders 0,015 Mol/l, verwendet.

Es ist auch bevorzugt, als Kupfer-Maßlösung eine Kupfersulfatlösung, insbesondere Kupfersulfatpentahydratlösung, zu verwenden. Insbesondere wird als Kupfersulfatpentahydratlösung eine solche mit einer Konzentration von 0,01 bis 0,02 Mol/l, ganz besonders 0,015 Mol/l, verwendet.

Zweckmäßigerweise wird das erfindungsgemäße Verfahren in der Weise durchgeführt, daß eine geeignete Menge der Probe mit einer Kupfer-Maßlösung bei einem pH-Wert von 8 bis 10 in Gegenwart eines Murexid-Indikators bis zur gelblichgrünen Farbe titriert wird und so ihr Gehalt an N-(Phosphonomethyl)-glycin + N-(Phosphonomethyl)-N-(carboxy-methyl)-glycin oder N-(Phosphonomethyl)-glycin + N,N-bis-(Phosphonomethyl)-glycin in an sich allgemein bekannter Weise berechnet wird, und danach ein anderer geeigneter Teil der Probe mit einer Wismut-Maßlösung bei einem pH-Wert von 1,5 bis 2,5 in Gegenwart eines Methylthymolblau-Indikators bis zur bläulichlila Farbe titriert wird und so in an sich allgemein bekannter Weise der Gehalt an N-(Phosphonomethyl)-N-(carboxy-methyl)-glycinoder N,N-bis-(Phosphonomethyl)-glycin berechnet wird. Die Differenz der beiden Messungen liefert den N-(Phosphonomethyl)-glycin-Gehalt.

Die Erfindung wird an Hand der folgenden Beispiele näher erläutert.

Beispiel 1

Analyse von technischem N-(Phosphonomethyl)-glycin

Es wurde von einer bis zur Gewichtskonstanz getrockneten Probe mit analytischer Genauigkeit eine Menge zwischen 200 und 300 mg in wenig Wasser und 2 ml einer Natriumhydroxydlösung mit einer Konzentration von 1 Mol/l gelöst, worauf in einem 100 ml Meßzylinder mit destilliertem Wasser bis zur Marke aufgefüllt wurde.

Ein Teil von 2 ml der Lösung wurde in einen Titrierkolben pipettiert und es wurden 100 ml Wasser, 1 ml einer Natriumhydroxydlösung mit einer Konzentration von 1 Mol/l, 10 ml einer Ammoniumchloridlösung mit einer Konzentration von 50 g/l sowie eine Spatelspitze eines Murexid-Mischindikators (1 g Murexid mit 300 g Kaliumnitrat gut homogenisiert) zugesetzt. Die Lösung wurde mit einer Kupfer-Maßlösung bis zur Erreichung der gelblichgrünen Farbe titriert. Aus dem Durchschnitt von 3 Messungen wurde der mittlere Verbrauch ($V_1$) berechnet.

Ein Teil von 50 ml der vorher bereiteten Stammlösung wurde in einen Titrierkolben pipettiert und es wurden 1 ml 10 gew.-%-ige Salpetersäure und eine Spatelspitze eines Methylthymolblau-Mischindikators (1 g Methylthymolblau mit 100 g Kaliumnitrat gut homogenisiert) zugesetzt. Die so erhaltene gelbe Lösung wurde mit der Wismut-Maßlösung bis zur Erreichung der bläulichlila Farbe titriert. Aus dem Durchschnitt von 3 Messungen wurde der mittlere Verbrauch ($V_2$) berechnet. In der folgenden Tabelle 2 sind die Analysen von 5 Proben zusammengefaßt. Infolge der Verfahrenstechnik muß in diesem Falle mit einer Verunreinigung durch N,N-bis-(Phosphonomethyl)-glycin(Molekulargewicht = 263,1) gerechnet werden. Der Faktor der Kupfer-Maßlösung war $f_1$ = = 1,0291 und der der Wismut-Maßlösung $f_2$ = 1,0032.

Tabelle 2

| Einwaage in mg | Verbrauch der $Cu^{+2}$-Maßlösung in ml | Verbrauch der $Bi^{+3}$-Maßlösung in ml | Korrigierter Verbrauch $f_1 \times Cu^{+2} - \frac{f_2 \times Bi^{+3}}{5}$ in ml | N,N-bis-(Phosphonomethyl)-glycin in Gew.-% | N-(Phosphonomethyl)-glycin in Gew.-% |
|---|---|---|---|---|---|
| 237,0 | 8,85 | 0,40 | 9,03 | 1,3 | 96,6 |
| 252,3 | 9,42 | 0,62 | 9,57 | 1,9 | 96,2 |
| 225,3 | 8,53 | 0,58 | 8,66 | 2,0 | 97,5 |
| 241,5 | 9,18 | 0,51 | 9,34 | 1,7 | 98,1 |
| 213,4 | 8,05 | 0,44 | 8,20 | 1,6 | 97,4 |

In der drittletzten vertikalen Spalte der Tabelle 2 handelt es sich um einen Zwischenrechnungswert, der in der Weise erhalten wird, daß der Faktor $f_1$ der Kupfer-Maßlösung mit dem Verbrauch der $Cu^{+2}$-Maßlösung (abgekürzt mit $Cu^{+2}$ bezeichnet) multipliziert wird und davon der 5-te Teil des Produktes des Faktors $f_2$ der $Bi^{+3}$-Maßlösung (abgekürzt mit $Bi^{+3}$ bezeichnet) und ihres Verbrauches subtrahiert wird (deswegen der 5-te Teil, weil zum Titrieren das 5-fache Lösungsvolumen verwendet wurde).

Der Gehalt an N,N-bis-(Phosphonomethyl)-glycin wurde in der folgenden allgemein bekannten Weise errechnet. Der Faktor $f_2$ der Wismut-Maßlösung (1,0032) wird mit dem Verbrauch der Wismut-Maßlösung (dritte vertikale Spalte der Tabelle 2), der Konzentration der Wismut-Maßlösung (welche sich aus der Herstellung der Wismut-Maßlösung als 0,015 Mol/l erwies), der Verdünnung (in diesem Beispiel war diese 2) und dem Molekulargewicht des N,N-bis-(Phosphonomethyl)-glycines (263,1) multipliziert und das so erhaltene Produkt wird durch die Einwaage (erste vertikale Spalte der Tabelle 2) dividiert und mit 100 multipliziert (um auf Prozente zu kommen).

Die Berechnung des Gehaltes an N-(Phosphonomethyl)-glycin erfolgte auf der Grundlage des korrigierten Verbrauches der drittletzten vertikalen Spalte der Tabelle 2. Es wurde der in der drittletzten vertikalen Spalte der Tabelle 2 stehende korrigierte Verbrauch mit der Konzentration der Kupfer-Maßlösung (0,015 Mol/l), der Verdünnung (10) und dem Molekulargewicht des N-(Phosphonomethyl)-glycines (169,1) multipliziert und das Produkt wurde durch die Einwaage (erste vertikale Spalte) dividiert und das ganze wurde mit 100 multipliziert.

Die Kupfer- und Wismut-Maßlösungen sind wie folgt bereitet worden.

I) Kupfer-Maßlösung

A) Herstellung der Kupfer-Maßlösung

Es wurden 3,6 g Kupfer(II)-sulfatpentahydrat in einem 1 l Meßkolben eingewogen und in destilliertem Wasser gelöst, worauf das Volumen der Lösung auf 1 l aufgefüllt wurde (Konzentration etwa 0,015 Mol/l).

B) Bestimmung des Wirkungswertes der Kupfer-Maßlösung

Es wurden 10,0 ml einer Äthylendiamintetraessigsäurelösung mit einer Konzentration von 0,01 Mol/l in einen Titrierkolben pipettiert und 100 ml Wasser, 1 ml einer Natriumhydroxydlösung mit einer Konzentration von 1 Mol/l, 10 ml einer Ammoniumchloridlösung mit einer Konzentration von 50 g/l und eine Spatelspitze eines Murexid-Mischindikators (1 g Murexid mit 300 g Kaliumnitrat gut homogenisiert) zugesetzt. Die Lösung wurde mit der Kupfer-Maßlösung bis zur Erreichung der gelblichgrünen Farbe titriert. Aus dem Durchschnitt von 3 Messungen wurde der mittlere Verbrauch ($V_3$) berechnet.

Faktor der Kupfer-Maßlösung:

$$f_1 = \frac{10 \times 0{,}01 \times f_3}{V_3 \times 0{,}015},$$

worin

$f_3$ der Faktor der Äthylendiamintetraessigsäurelösung ist.

II) Wismut-Maßlösung

A) Herstellung der Wismut-Maßlösung

Es wurden 4,36 g basisches Wismutnitrat [4 $BiNO_3(OH)_2$ . BiOH] in 10 ml konzentrierter Salpetersäure und wenig Wasser gelöst, worauf das Volumen der Lösung mit 5 ml konzentrierter Salpetersäure angesäuertem Wasser vorsichtig auf 1 l aufgefüllt wurde (Konzentration: etwa 0,015 Mol/l).

B) Bestimmung des Wirkungswertes der Wismut-Maßlösung

Es wurden 10,0 ml einer Äthylendiamintetraessigsäurelösung mit einer Konzentration von 0,01 Mol/l in einen Titrierkolben pipettiert, 30 bis 40 ml Wasser, 0,5 ml einer 10 gew.-%-igen Salpetersäure und eine Spatelspitze eines Methylthymolblau-Mischindikators (1 g Methylthymolblau mit 100 g Kaliumnitrat gut homogenisiert) zugesetzt. Die Lösung wurde mit der Wismut-Maßlösung bis zum Erreichen der bläulichlila Farbe titriert. Aus dem Durchschnitt von 3 Messungen wurde der mittlere Verbrauch ($V_4$) berechnet.

Faktor der Wismut-Maßlösung:

$$f_2 = \frac{10{,}0 \times 0{,}01 \times f_4}{V_4 \times 0{,}015} ,$$

worin

$f_4$ der Faktor der Äthylendiamintetraessigsäurelösung ist.

Beispiel 2

Analyse von zusammengewogenem N-(Phosphonomethyl)-glycin und N-(Phosphonomethyl)-N-(carboxy-methyl)-glycin

Es wurden aus Standard-N-(Phosphonomethyl)-glycin (Molekulargewicht = 169,1) und Standard-N-(Phosphonomethyl)-N-(carboxy-methyl)-glycin (Molekulargewicht = = 227,1) Mischungen verschiedener Zusammensetzung hergestellt und nach dem Beispiel 1 vorgegangen.

Die Ergebnisse sind in der folgenden Tabelle 3 zusammengestellt.

Tabelle 3

| Einwaage in mg | N-(Phosphonomethyl)-N-(carboxymethyl)-glycin in der Einwaage in Gew.-% | Verbrauch der $Cu^{+2}$-Maßlösung in ml | Verbrauch der $Bi^{+3}$-Maßlösung in ml | Korrigierter Verbrauch $f_1 \times Cu - \frac{f_2 \times Bi^{+3}}{5}$ in ml | N-(Phosphonomethyl)-N-(carboxymethyl)-glycin in Gew.-% | N-(Phosphonomethyl)-glycin in Gew.-% |
|---|---|---|---|---|---|---|
| 243,5 | 1,0 | 9,29 | 0,34 | 9,49 | 0,95 | 98,86 |
| 223,7 | 2,0 | 8,53 | 0,64 | 8,65 | 1,96 | 98,08 |
| 232,4 | 5,0 | 8,79 | 1,76 | 8,69 | 5,17 | 94,85 |
| 218,9 | 10,0 | 8,15 | 3,24 | 7,74 | 10,11 | 89,69 |
| 236,1 | 20,0 | 8,62 | 7,00 | 7,47 | 20,26 | 80,25 |

Die Berechnung erfolgte wie im Beispiel 1 beschrieben, jedoch unter Verwendung der entsprechenden Molekulargewichte.

Die Kupfer- und Wismut-Maßlösungen sind wie am Ende des Beispieles 1 beschrieben hergestellt worden.

Beispiel 3

Verfolgen der Oxidation von N-(Phosphonomethyl)-N-(carboxy-methyl)-glycin

Dem Oxydationsgemisch wurden in bestimmten Zeitabständen Proben entnommen, es wurde durch Filterpapier filtriert und passende Mengen wurden titriert. Es wurde wie im Beispiel 1 beschrieben vorgegangen, jedoch mit dem Unterschied, daß das Volumen der Titrierlösungen mit destilliertem Wasser im Falle der Kupfer-Maßlösung auf etwa 100 ml und im Falle der Wismut-Maßlösung auf etwa 50 ml eingestellt wurde. Wenn nur mit Wismut titriert wurde, dann wurde nur der Verbrauch des N-(Phosphonomethyl)-N-(carboxy-methyl)-glycines verfolgt, beim Titrieren mit Kupfer konnte auch die N-(Phosphonomethyl)-glycin-Bildung verfolgt werden. Die Verminderung der Summe von N-(Phosphonomethyl)-glycin und N-(Phosphonomethyl)-N-(carboxy-methyl)-glycin weist auf den Ablauf von Nebenreaktionen hin. In diesem Beispiel werden die Ergebnisse des mit unveränderter Probemenge durchgeführten Verfolgens der Reaktion gezeigt. Sie sind in der folgenden Tabelle 4 zusammengestellt.

Tabelle 4

| Oxydationszeit in Stunden | $f_1 \times Cu^{+2}$ in ml | $f_2 \times Bi^{+3}$ in ml | N-(Phosphonomethyl)-N-(carboxy-methyl)-glycin in Mol% | N-(Phosphonomethyl)-glycin in Mol% | N-(Phosphonomethyl)-N-(carboxy-methyl)-glycin + N-(Phosphonomethyl)-glycin in Mol% |
|---|---|---|---|---|---|
| 0 | 15,1 | 15,1 | 100,0 | 0,0 | 100,0 |
| 0,5 | 15,0 | 12,9 | 85,4 | 13,9 | 99,3 |
| 1,0 | 14,9 | 10,8 | 71,5 | 27,2 | 98,7 |
| 1,5 | 14,9 | 8,6 | 57,0 | 41,7 | 98,7 |
| 2,0 | 14,7 | 6,6 | 43,7 | 53,6 | 97,3 |
| 2,5 | 14,5 | 4,5 | 29,8 | 66,2 | 96,0 |
| 3,0 | 14,4 | 2,4 | 15,9 | 79,5 | 95,4 |
| 3,5 | 14,1 | 0,3 | 2,0 | 91,4 | 93,4 |

Die Berechnungen erfolgten analog wie im Beispiel 1.

Die Wismut- und Kupfer-Maßlösungen sind wie am Ende des Beispieles 1 beschrieben hergestellt worden.

Beispiel 4

Analyse von N-(Phosphonomethyl)-glycin-Natrium in Zubereitung mit einer Konzentration von 200 g/l

Von der Zubereitung wurden 10,0 ml in einen 1 l Meßzylinder pipettiert und mit destilliertem Wasser bis zur Marke aufgefüllt. Von dieser Stammlösung wurde in der im Beispiel 1 beschriebenen Weise der Gehalt an N-(Phosphonomethyl)-glycin beziehungsweise je nach der Verfahrenstechnik an N-(Phosphonomethyl)-N-(carboxy-methyl)-glycin beziehungsweise N,N-bis-(Phosphonomethyl)-glycin bestimmt.

In der folgenden Tabelle 5 sind die Ergebnisse von 5 Produktionschargen angegeben und zum Vergleich erscheinen auch die Ergebnisse von 5 Produktionschargen, welche auf dem Wege des Nitrosierens erhalten wurden.

Tabelle 5

| Probe | N,N-bis-(Phosphonomethyl)-glycin in g/l | N-(Phosphonomethyl)-glycin [Komplex] in g/l | N-(Phosphonomethyl)-glycin [Nitrosieren] in g/l |
|---|---|---|---|
| 1 | 3,2 | 198,2 | 202,7 |
| 2 | 2,8 | 203,4 | 200,1 |
| 3 | 3,6 | 201,7 | 203,9 |
| 4 | 3,1 | 197,9 | 195,0 |
| 5 | 2,4 | 201,3 | 199,5 |

In der obigen Tabelle 5 wurden keine Teildaten, wie Verbrauch und Einwaage, angegeben, sondern nur die den obigen Beispielen entsprechend errechneten Endergebnisse. Der Zweck dieser Tabelle ist, die nach dem erfindungsgemäßen Verfahren und nach dem aus dem Stand der Technik bekannten Nitrosierverfahren erhaltenen Ergebnisse zu vergleichen.

Die Kupfer- und Wismut-Maßlösungen sind wie am Ende des Beispieles 1 beschrieben erhalten worden.

Beispiel 5

Analyse von technischem N-(Phosphonomethyl)-N-(carboxy-methyl)-glycin beziehungsweise N,N-bis-(Phosphonomethyl)-glycin

Von einer bis zur Gewichtskonstanz getrockneten Probe wurden genau Mengen um 250 beziehungsweise 300 mg eingewogen, in wenig Wasser und 2 ml einer Natriumhydroxydlösung mit einer Konzentration von 1 Mol/l gelöst, worauf in einem 100 ml Meßzylinder mit destilliertem Wasser bis zur Marke aufgefüllt wurde. Ein Anteil von 10 ml der so erhaltenen Stammlösung wurde in einen Titrierkolben pipettiert, es wurden etwa 40 ml destilliertes Wasser, 1 ml einer 10 gew.-%-igen Salpetersäure und eine Spatelspitze eines mit Methylthymolblau-Mischindikators (1 g Methylthymolblau mit 100 g Kaliumnitrat gut homogenisiert) zugesetzt, worauf mit einer Wismut-Maßlösung bis zum Erreichen der bläulichlila Farbe titriert wurde. Aus dem Verbrauch wurde in an sich bekannter Weise der Wirkstoffgehalt berechnet.

In der folgenden Tabelle 6 sind die von 5 parallelen Titrationen von je 1 Probe errechneten Werte zusammengestellt.

## Tabelle 6

| Probe | N-(Phosphonomethyl)-N-(carboxy-methyl)-glycin in Gew.-% | N,N-bis-(Phosphonomethyl)-glycin in Gew.-% |
|---|---|---|
| 1 | 98,2 | 97,7 |
| 2 | 98,7 | 98,5 |
| 3 | 98,6 | 98,3 |
| 4 | 98,0 | 98,1 |
| 5 | 98,4 | 98,7 |

Auch in der Tabelle 6 erscheinen nur die Endergebnisse, deren Berechnung in der im Beispiel 1 angegebenen Weise erfolgte.

Die Wismut-Maßlösung ist wie am Ende des Beispieles 1 beschrieben hergestellt worden.

Beispiel 6

Analyse von N,N-bis-(Phosphonomethyl)-glycin in 85 gew.-%-iger Zubereitung

Von der jeweiligen Probe wurden genau Mengen um 400 mg eingewogen und es wurde wie im Beispiel 5 beschrieben vorgegangen.

Die Wirkstoffgehalte von 5 Proben sind in der folgenden Tabelle 7 zusammengestellt.

Tabelle 7

| Probe | 1 | 2 | 3 | 4 | 5 |
|---|---|---|---|---|---|
| N,N-bis-(Phosphonomethyl)-glycin in Gew.-% auf Gewicht | 85,2 | 87,1 | 88,3 | 86,4 | 86,6 |

Auch in der Tabelle 7 erscheinen nur die Endergebnisse, deren Berechnung in der im Beispiel 1 angegebenen Weise erfolgte.

Die Wismut-Maßlösung ist wie am Ende des Beispieles 1 beschrieben hergestellt worden.

## Patentansprüche

**1.** Verfahren zur selektiven quantitativen Bestimmung von N-(Phosphonomethyl)-glycin, N-(Phosphonomethyl)-N-(carboxy-methyl)-glycin, N,N-bis-(Phosphonomethyl)-glycin beziehungsweise einer Mischung von N-(Phosphonomethyl)-N-(carboxy-methyl)-glycin und N-(Phosphonomethyl)-glycin oder einer Mischung von N-(Phosphonomethyl)-glycin und N,N-bis-(Phosphonomethyl)-glycin, dadurch gekennzeichnet, daß man

a) mit einer Wismut-Maßlösung den Gehalt an N-(Phosphonomethyl)-N-(carboxy-methyl)-glycin beziehungsweise N,N-bis-(Phosphonomethyl)-glycin bei einem pH-Wert von 1,5 bis 2,5 in Gegenwart eines Methylthymolblau-Indikators durch komplexometrische Titration bestimmt und

b) mit einer Kupfer-Maßlösung die Summe des Gehaltes an N-(Phosphonomethyl)-glycin und N-(Phosphonomethyl)-N-(carboxy-methyl)-glycin oder N-(Phosphonomethyl)-glycin und N,N-bis-(Phosphonomethyl)-glycin bei einem pH-Wert von 8 bis 10 in Gegenwart eines Murexidindikators durch komplexometrische Titration bestimmt

sowie gegebenenfalls aus den Ergebnissen beider Bestimmungen den Gehalt der Probe an N-(Phosphonomethyl)-glycin berechnet.

**2.** Verfahren nach Anspruch 1 a), dadurch gekennzeichnet, daß man als Wismut-Maßlösung eine basische Wismutnitratlösung in Salpetersäure verwendet.

**3.** Verfahren nach Anspruch 1 a) oder 2, dadurch gekennzeichnet, daß man als basische Wismutnitratlösung eine solche mit einer Konzentration von 0,01 bis 0,02 Mol/l, insbesondere 0,015 Mol/l, verwendet.

**4.** Verfahren nach Anspruch 1 b), dadurch gekennzeichnet, daß man als Kupfer-Maßlösung eine Kupfersulfatlösung, insbesondere Kupfersulfatpentahydratlösung, verwendet.

**5.** Verfahren nach Anspruch 1 b) oder 4, dadurch gekennzeichnet, daß man als Kupfersulfatpentahydratlösung eine solche mit einer Konzentration von 0,01 bis 0,02 Mol/l, insbesondere 0,015 Mol/l, verwendet.

## Claims

**1.** Method for selective determination of N-(phosphonomethyl)-glycine, N-(phosphonomethyl)-N-(carboxy-methyl)-glycine, N,N-bis-(phosphonomethyl)-glycine and respectively of a mixture of N-(phosphonomethyl)-N-(carboxy-methyl)-glycine and N-(phosphonomethyl)-glycine or a mixture of N-(phosphonomethyl)-glycine and N,N-bis-(phosphonomethyl)-glycine characterized in that

a) the content of N-(phosphonomethyl)-N-(carboxy-methyl)-glycine and N,N-bis-(phosphonomethyl)-glycine respectively is determined with a bismuth standard solution at a pH value of 1,5 to 2,5 in the presence of a methylthymol blue indicator by way of complexometric titration; and

b) the sum of the content of N-(phosphonomethyl)-glycine and N-(phosphonomethyl)-N-(carboxy-methyl)-glycine or N-(phosphonomethyl)-glycine and N,N-bis-(phosphonomethyl)-glycine is determined with a copper standard solution at a pH value of 8 to 10 in the presence of a murexide indicator by way of complexometric titration;

and optionally the sample content of N-(phosphonomethyl)-glycine is calculated from the results of both determinations.

**2.** Method according to claim 1 a), characterized in that a basic bismuth nitrate solution in nitric acid is used as bismuth standard solution.

**3.** Method according to claim 1 a) or 2, characterized in that such a concentration of 0,01 to 0,02 mole/l, particularly 0,015 mole/l, is used as basic bismuth nitrate solution.

**4.** Method according to claim 1 b) characterized in that a copper sulfate solution, particularly a copper sulfate pentahydrate solution, is used as copper standard solution.

**5.** Method according to claim 1 b) or 4 characterized in that such a concentration of 0,01 to 0,02 mole/l, particularly 0,015 mole/l, ist used as copper sulfate pentahydrate solution.

**Revendications**

**1.** Procédé de détermination quantitative et sélective de N-(phosphonométhyle)-glycine, N-(phosphonométhyle)-N-(carboxyméthyle)-glycine, N,N-bis-(phosphonométhyle)-glycine, et aussi d'un mélange de N-(phosphonométhyle)-N-(carboxyméthyle)-glycine et N-(phosphonométhyle)-glycine, ou d'un mélange de N-(phosphonométhyle)-glycine et N,N-bis-(phosphonométhyle)-glycine, se caractérisant comme suit :

a) au moyen d'une liqueur titrée de bismuth, détermination par titrage complexométrique, de la teneur en N-(phosphonométhyle)-N-(carboxyméthyle)-glycine et aussi N,N-bis-(phosphonométhyle)-glycine, avec une valeur pH de 1,5 à 2,5 et en présence d'un indicateur bleu de méthylethymole, et

a) au moyen d'une liqueur titrée de cuivre, détermination par titrage complexométrique, de la somme de la teneur en N-(phosphonométhyle)-glycine et N-(phosphonométhyle)-N-(carboxyméthyle)-glycine ou N-(phosphonométhyle)-glycine et N,N-bis-(phosphonométhyle)-glycine, avec une valeur pH de 8 à 10 et en présence d'un indicateur murexide, et

à partir du résultat des deux déterminations, le calcul de la teneur de l'échantillon en N-(phosphonométhyle)-glycine.

**2.** Procédé selon la revendication 1 a), caractérisé par le fait qu'on ulitise comme liqueur titrée de bismuth, une solution basique de nitrate de bismuth dans de l'acide nitrique.

**3.** Procédé selon la revendication 1 a) ou 2, caractérisé par le fait qu'on ulitise pour la solution basique de nitrate de bismuth une concentration de 0,01 à 0,02 mol/l, et en particulier 0,015 mol/l.

**4.** Procédé selon la revendication 1 b), caractérisé par le fait qu'on ulitise comme solution titrée de cuivre, une solution de sulfate de cuivre, en particulier une solution de pentahydrate de sulfate de cuivre.

**5.** Procédé selon la revendication 1 b) ou 4, caractérisé par le fait qu'on ulitise pour la solution de pentahydrate de sulfate de cuivre une concentration de 0,01 à 0,02 mol/l, et en particulier 0,015 mol/l.